# EUROPÄISCHE PATENTSCHRIFT

(11) **EP 0 872 643 B1**
(45) Veröffentlichungstag und Bekanntmachung des Hinweises auf die Patenterteilung: **28.05.2003**
(21) Anmeldenummer: 98104204.7
(22) Anmeldetag: 10.03.1998
(51) Int. Cl.: F04D 29/42, F04D 29/66

(54) **Luftzuführung für Gebläse**
Air inlet for a fan
Entrée d'air pour une soufflante

(30) Priorität: 15.04.1997 DE 19715581
(43) Veröffentlichungstag der Anmeldung: 21.10.1998
(73) Patentinhaber: GOTTLIEB WEINMANN GERÄTE FÜR MEDIZIN UND ARBEITSSCHUTZ GMBH & CO., 22525 Hamburg (DE)
(72) Erfinder: Feldhahn, Karl-Andreas, Dr., 22761 Hamburg (DE); Paesch, Rainer, 25451 Quickborn (DE)
(74) Vertreter: Klickow, Hans-Henning

(56) Entgegenhaltungen:
- EP-A- 0 740 080
- WO-A-94/22064
- DE-A- 1 403 576
- DE-A- 19 510 553
- GB-A- 2 259 327

## Beschreibung

Die Erfindung betrifft eine Luftzuführung für Gebläse von Geräten zur Beatmung, wie zur Behandlung der Schlafapnoe, wobei unter Zwischenschaltung des Gebläses ein Luftzuführkanal mit einem Luftausgang verbunden ist, sowie bei der der Luftzuführkanal in mindestens zwei Teilkanäle aufgeteilt ist, die sich wenigstens über einen Teil der Längsausdehnung des Luftzuführkanals in Richtung der Luftströmung erstrecken.

Bei der Verwendung von Gebläsen zur Luftförderung für Nasalmasken besteht ein erhebliches Problem darin, daß aufgrund der Luftströmung Geräusche erzeugt werden. Diese Geräusche sind vor allem dann störend, wenn diese im medizinischen Bereich, wie bei Nasalmasken, eine Verwendung in der unmittelbaren Umgebung des Körpers eines Patienten erfolgt. Eine Verschärfung der Problemstellung tritt dann auf, wenn die Geräte im Rahmen einer Beatmungstherapie eingesetzt werden, die während des Schlafes eines Patienten durchgeführt oder fortgesetzt wird. Bei einer wesentlichen Geräuschentwicklung kann hierdurch sowohl der Schlaf des Patienten als auch der Schlaf von weiteren in seiner Umgebung ruhenden Personen gestört werden.

Im Bereich des Lufteinlasses von Gebläsen, die bei derartigen Anwendungen eingesetzt werden, können Luftfilter eingesetzt werden, die ebenfalls zu einer gewissen Geräuschdämpfung führen. Es ist jedoch durch diese Maßnahme bislang nicht möglich, eine angestrebte erhebliche Geräuschreduzierung zu realisieren.

Aus der EP-A-0 740 080 D1 ist es bekannt, im Bereich einer Luftzuführung eines Verdichters gekrümmt verlaufende Luftzuführkanäle anzuordnen. Mehrere Teilkanäle werden relativ zueinander von Wandelementen getrennt, so daß sich die Teilkanäle über wenigstens einen Teil einer Längsausdehnung des Luftzufuhrkanals in Richtung der Luftströmung erstrecken. Die bereitgestellten Teilkanäle dienen sowohl zur Schalldämpfung als auch zur Luftfilterung.

Aus der GB 2 079 373 A ist es bekannt, im Bereich der Einströmöffnung eines Gebläses einen luftdurchlässigen Dämpfer anzuordnen. In einer anderen Ausführungsform wird ein luftdurchlässiges Dämpfungselement im Eingangsbereich eines Gebläses in der GB 2 260 576 erläutert.

Aus der DE-OS 22 54 650 ist ein Beatmungsgerät mit Gebläse bekannt, bei dem die Luftförderung durch eine Turbine vorgenommen wird, die von einem Elektromotor angetrieben ist. Eine Schalldämpfung im Bereich der Einströmöffnung ist nicht vorgesehen.

Das DE-GM 1 832 485 erläutert die Verwendung von Luftleitblechen im Bereich von Strömungskanälen. Insbesondere werden die Luftleitelemente zur Reduktion des Strömungswiderstandes im Bereich von Rohrbögen des Strömungskanals verwendet.

Aus der CH-PS 155 868 ist ebenfalls die Verwendung eines Strömungsleitbleches im Bereich eines Leitungsbogens bekannt.

Die DE 29 36 666 A1 erläutert die Verwendung einer Geräuschdämmung im Bereich der Zuströmseite eines Gebläses. Insbesondere wird die Beschichtung von Flächen, die der Strömung ausgesetzt sind, mit einem schalldämmenden Material erwähnt.

Aus der US 55 26 805 ist die Verwendung eines geschäumten Materials zur Innenauskleidung eines Rohres bekannt. Das Rohr weist an der für die Innenverkleidung vorgesehenen Stelle eine Querschnittserweiterung auf, so daß ein freier Strömungsquerschnitt auch im Bereich der Umfassung des Strömungsweges mit dem geschäumten Material bereitgestellt wird.

Das DE 86 25 027 U1 zeigt eine äußere Ummantelung eines Rohres mit einem Sauerstoff. Die Ummantelung dient als Luftfilter, um Schadstoffpartikel zurückzuhalten.

In der britischen Druckschrift GB 2 209 474 wird ein Atemfilter beschrieben, der ein Gebläse aufweist und der zur Verwendung in Kombination mit einer Gasmaske vorgesehen ist. Im Einströmbereich ist ein großflächiges und voluminöses Filtermaterial angeordnet, um Luftverunreinigung zurückzuhalten. Ausgehend vom Filter verjüngt sich ein Strömungsraum in Richtung auf das Lüfterrad. Im Bereich einer Vorbeiführung der Luftströmung am Lüftermotor wird über tangential nach außen weisende Stege eine Luftverwirbelung und Durchmischung unterstützt.

Aufgabe der vorliegenden Erfindung ist es, eine Vorrichtung der einleitend genannten Art auf einfache Weise derart zu verbessern, daß die Geräuschemissionen vermindert werden.

Diese Aufgabe wird erfindungsgemäß dadurch gelöst, daß sich der Luftzuführkanal mindestens bereichsweise relativ zu einem Zentralbereich spiralförmig erstreckt und daß eine Ausströmöffnung in einem Zentralbereich des Luftleitelementes derart angeordnet ist, daß die Ausströmöffnung ein einen zentralen Lüfterraum eines Lüfterrades des Gebläses mündet.

Ergänzend ist es möglich, daß der Luftzuführkanal mit einem luftdurchlässigen Material zur Bildung einer Vielzahl von Durchlässen in der Art von Teilkanälen ausgefüllt ist.

Aufgrund der durch die Realisierung der Teilkanäle verursachten Laminarisierung der Luftströmung können Verwirbelungen weitgehend vermieden beziehungsweise reduziert werden. Es zeigt sich, daß ein erheblicher Anteil der Geräuschemissionen durch derartige Verwirbelungen erzeugt wird. Die Vermeidung der Verwirbelung hat darüber hinaus den Vorteil, daß ein auftretender Strömungswiderstand reduziert wird. Die Geräteakzeptanz derartige Ausbildungen nutzender Patienten kann somit erheblich verbessert werden.

Zur Bereitstellung einer raumsparenden Anordnung ist vorgesehen, daß sich der Zuführkanal mindestens bereichsweise relativ zu einem Zentralbereich spiralförmig erstreckt.

Eine flache Bauform kann dadurch realisiert werden, daß mindestens drei Teilkanäle in einer räumlichen Richtung nebeneinander angeordnet sind.

Eine weitere Laminisierung der Luftströmung kann dadurch unterstützt werden, daß die Teilkanäle in zwei unterschiedlichen räumlich getrennten Ebenen angeordnet sind.

Eine weitere Maßnahme zur Vergleichmäßigung der Luftströmung besteht darin, daß die Teilkanäle mindestens bereichsweise ein poröses Material aufnehmen.

Eine bevorzugte Materialauswahl besteht darin, daß die Teilkanäle als poröses Material ein grob gesintertes Material aufnehmen.

Ein geringes Baugewicht kann dadurch unterstützt werden, daß die Teilkanäle als poröses Material einen Schaumstoff aufnehmen.

Zur weiteren Vergleichmäßigung der Strömungsführung ist auch daran gedacht, daß die Teilkanäle mindestens teilweise im Eintrittsbereich eine Querschnittserweiterung aufweisen.

In der Zeichnung ist ein Ausführungsbeispiel der Erfindung schematisch dargestellt. Es zeigen:
- Fig. 1: einen Querschnitt durch eine Luftführung,
- Fig. 2: eine perspektivische Darstellung der geschnittenen Luftführung gemäß Fig. 1,
- Fig. 3: einen Querschnitt gemäß Schnittlinie III-III in Fig. 1 durch eine komplette Gebläseeinheit,
- Fig. 4: einen Querschnitt durch eine andere Ausführungsform mit modifiziertem Verlauf der Strömungskanäle und
- Fig. 5: eine Seitenansicht eines Gebläses mit Lüfterrad, von dem das spiralförmige Luftleitelement abgenommen ist.

Bei der dargestellten Anordnung ist ein Luftleitelement (1) für eine Luftzuführung durch eine Außenwandung (2) mit einer nicht gezeigten Abdeckung eines Luftzuführkanals (3) begrenzt. Der Luftzuführkanal (3) ist von Einbauelementen (4) als Zwischenwände in Teilkanäle (5) unterteilt. Die Teilkanäle (5) verbinden eine Einströmöffnung (6) mit einer Ausströmöffnung (7) in einem Zentralbereich (10)

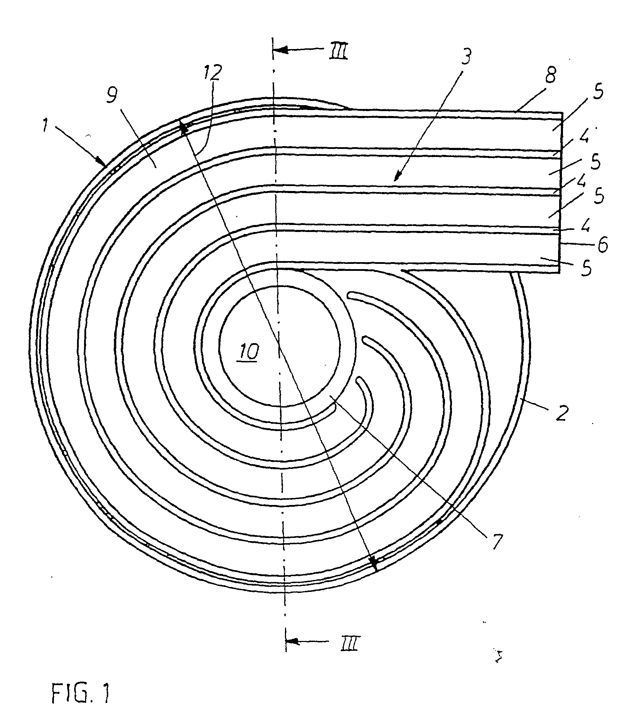

Bei der in Fig. 1 dargestellten Ausführungsform ist die Einströmöffnung (6) im Bereich eines Endstutzens (8) angeordnet, der im wesentlichen linear verläuft und in einen spiralartigen Fortsetzungsbereich (9) einmündet. Der Zentralbereich (10) des Luftleitelementes (1) wird typischerweise mit einem steuerbaren Gebläse (13) angekoppelt.

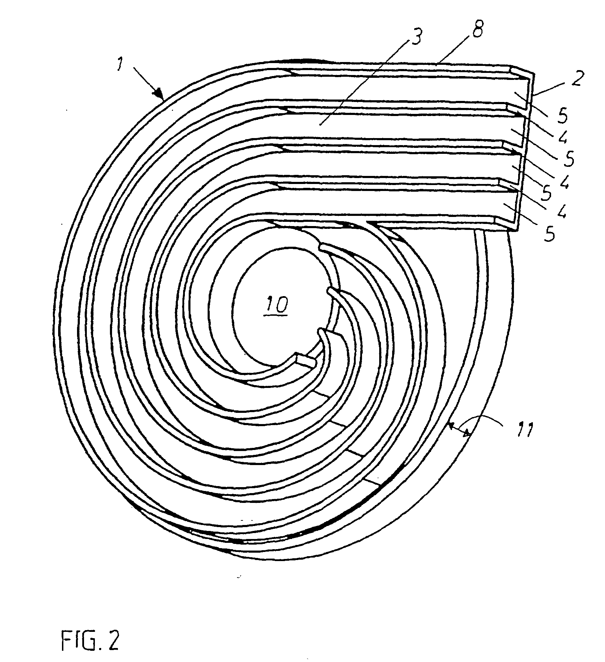

Fig. 2 zeigt das Luftleitelement gemäß Fig. 1 in einer perspektivischen Darstellung. Es ist hieraus insbesondere erkennbar, daß das Luftleitelement (1) eine scheibenartige Ausbildung aufweist und daß die Teilkanäle (5) als näherungsweise spiralförmige Hohlräume innerhalb des scheibenartigen Körpers verlaufen. Eine Scheibendicke (11) ist wesentlich geringer als ein Scheibendurchmesser (12) ausgebildet. Durch die relativ geringe Scheibendicke (11) ist es problemlos möglich, das Luftleitelement (1) als Zusatzelement im Bereich einer Zuleitöffnung oder einer Ableitöffnung eines Gebläses (13) anzuordnen.

Es ist ebenfalls erkennbar, daß die Teilkanäle (5) in radialer Richtung nebeneinander angeordnet sind. Grundsätzlich ist es ebenfalls denkbar, bezüglich der dargestellten Orientierung eine Anordnung von Teilkanälen (5) übereinander vorzunehmen oder durch eine Anordnung der Teilkanäle (5) sowohl übereinander als auch nebeneinander ein zweidimensionales Feld von Teilkanälen (5) bereitzustellen.

Zusätzliche Varianten zur Ausbildung bestehen in der Verwendung von porösen Materialien, grobgesinterten Materialien beziehungsweise von Schaumstoff, als Einlagen in die Teilkanäle (5). Es können gleichfalls Kombinationen unterschiedlicher Materialien eingesetzt werden. Zur Durchführung einer weiteren Vergleichmäßigung der Luftströmung ist es möglich, die Öffnungen (6,7) mit Aufweitungen zu versehen.

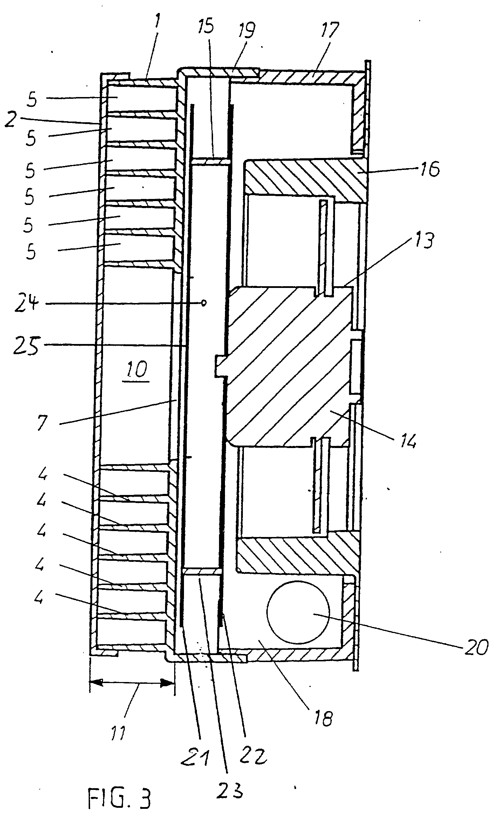

Fig. 3 zeigt durch die Schnittdarstellung die Kombination des Luftleitelementes (1) mit einem Gebläse (13), das einen Motor (14) sowie ein Lüfterrad (15) aufweist. Der Motor (14) wird von einer Motoraufnahme (16) getragen, die ihrerseits von einem Gebläseunterteil (17) aufgenommen ist. Zwischen dem Motor (14) und der Motoraufnahme (16) erstreckt sich ein Strömungsraum (18), der einen Luftausgang (20) aufweist. Der Luftausgang ist über einen Schlauch mit der Nasalmaske für den Patienten verbunden.

Das Luftleitelement (1) ist mit einem Montageflansch (19) versehen, der mit dem Gebläseunterteil (17) derart zusammengefügt werden kann, daß sowohl eine mechanische Halterung als auch eine zuverlässige Strömungsführung gesichert ist. Der Zentralbereich (10) des Luftleitelementes (1) mündet zunächst in einen Lüfterraum , der seinerseits mit dem Strömungsraum (18) in Verbindung steht und über den Luftausgang (20) den Patienten versorgt.

Das Lüfterrad (15) besteht typischerweise aus zwei zueinander parallel angeordneten Tragplatten (21,22), die im Bereich ihrer Umfangsbegrenzung Fördersegmente (23) haltern. Typischerweise verlaufen die Fördersegmente (23) plattenartig tangential zur Umfangsbegrenzung der Tragplatten (21,22). Darüber hinaus weisen die Fördersegmente (23) einen Abstand zueinander auf. Bei einer Rotation des Lüfterrades (15) wird hierdurch eine Luftströmung aus einem Lüfterradinnenraum (24) heraus erzeugt.

Die der Ausströmöffnung (7) zugewandte Tragplatte (21) ist in einem mittleren Bereich mit einer Einströmöffnung (25) versehen. Insbesondere ist auch daran gedacht, die Fördersegmente (23) in Richtung auf den Lüfterradinnenraum (24) nach innen versetzt mit einem Abstand zum äußeren Umfang der Tragplatten (21,22) anzuordnen.

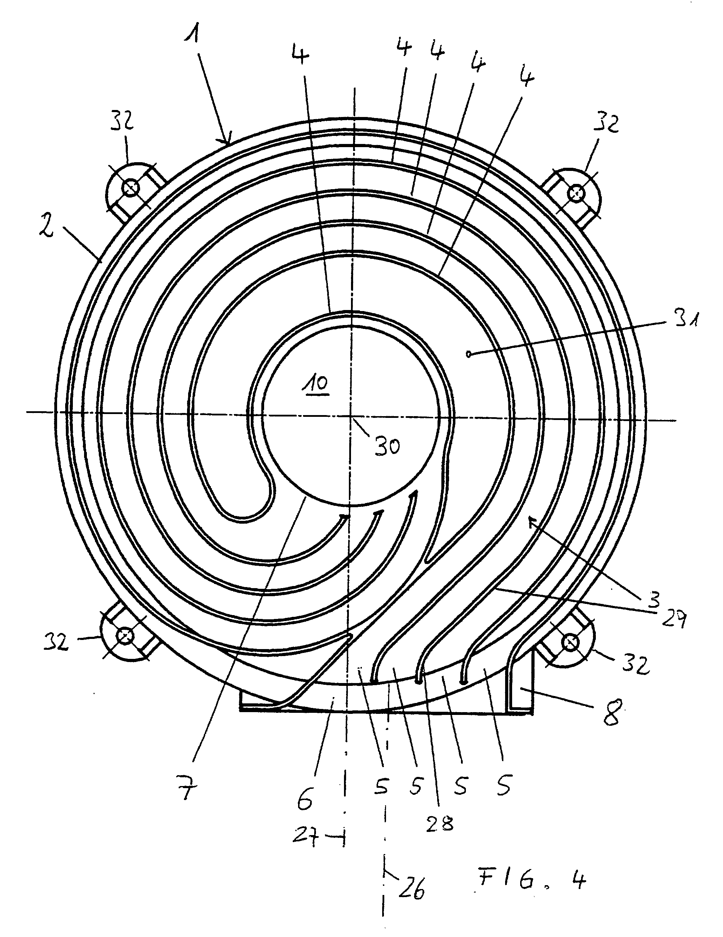

Fig. 4 zeigt eine gegenüber der Ausführungsform in Fig. 1 modifizierte Konstruktion des Luftleitelementes (1). Zunächst ist der Endstutzen (8) deutlich verkürzt ausgebildet. Es zeigt sich, daß sich in Kombination mit einem modifizierten Verlauf der Teilkanäle (5) auch bei einem verkürzten Endstutzen (8) eine gute Geräuschdämmung erreichen läßt.

Gegenüber der Ausführungsform in Fig. 1 erstreckt sich der Endstutzen (8) auch nicht als tangentiale Verlängerung der Außenwandung (5), sondern der Endstutzen (8) ist mit einer Stutzenmittellinie (26) in Richtung auf eine Dämpfermittellinie (27) versetzt. Es liegt allerdings weiterhin ein Abstand zwischen den Mittellinien (26,27) vor. Zur Überleitung der Teilkanälte (5) in den Endstutzen (8) sind die Einbauelemente (4) im Bereich ihrer in den Endstutzen (8) hineinragenden Enden mit Umleitungen (28) versehen, die die in Richtung der Stutzenmittellinie (26) einströmende Luft in den gekrümmten Verlauf der Teilkanäle (5) überleiten.

Gemäß einer besonderen Ausführungsvariante ist daran gedacht, zwischen den Umleitungen (28) und den gekrümmt verlaufenden Bereichen der Teilkanäle (5) näherungsweise linear verlaufende Wandteile (29) anzuordnen. Durch diese konstruktive Realisierung wird erreicht, daß die Teilkanäle (5) in einem überwiegenden Bereich ihrer Ausdehnung konzentrisch zu einem Mittelpunkt (30) des Luftleitelementes (1) verlaufen. Erst in einem Übergangsbereich zur Auströmöffnung (7) verlaufen dann die Einbauelemente (4) mit einer spiralartigen Krümmung. Auch in diesem Bereich mit spiralartiger Krümmung der Einbauelemente (4) ist aber daran gedacht, eine im wesentlichen konstante Querschnittdimensionierung der überwiegenden Anzahl der Teilkanäle (5) vorzusehen.

Aufgrund der bei einer Überleitung in den Zentralbereich (10) zu erwartenden Strömungsturbulenzen im Bereich des Teilkanales (5) mit dem geringsten Krümmungsradius relativ zum Mittelpunkt (30) erweitert sich dieser innenliegende Teilkanal (5) im Bereich seiner Überleitung in die Ausströmöffnung (7). Insbesondere ist daran gedacht, eine im wesentlichen kontinuierlich verlaufende trichterartige Aufweitung vorzusehen.

Zur Vermeidung relativ geringer Krümmungsradien der Teilkanäle (5) kann die Ausströmöffnung (7) des Luftleitelementes (1) bereichsweise von einem Distanzhohlraum (31) umschlossen sein, der von Bereichen der Einbauelemente (4) begrenzt ist. Zur Verbindung des Luftleitelementes (1) mit dem Gebläseunterteil (17) sind Befestigungsstege (32) vorgesehen, die beispielsweise Schrauben zur Verbindung mit dem Gebläseunterteil (17) haltern. Die Befestigungsstege (32) können zungenartig in radialer Richtung nach außen über die Außenwandung (2) überstehen.

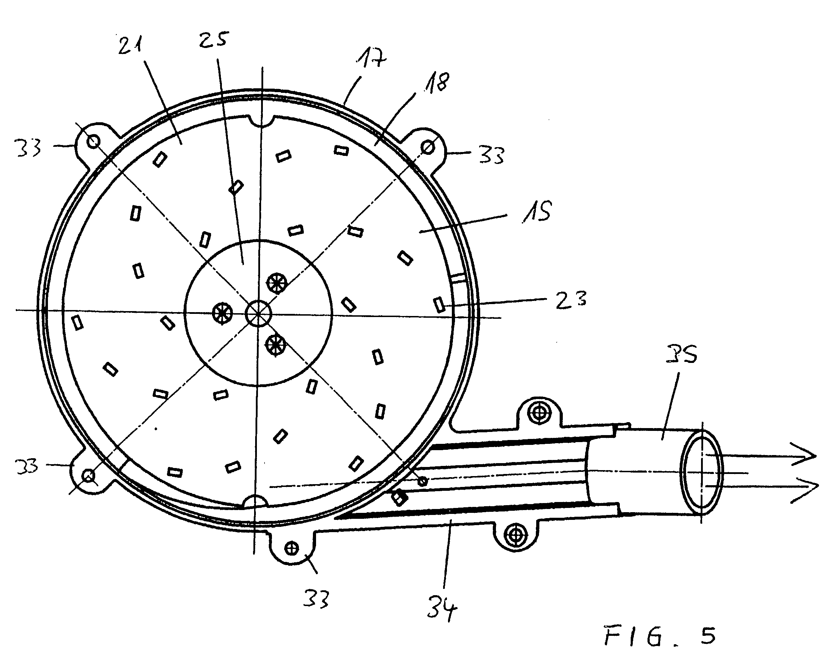

Aus Fig. 5 ist insbesondere die Einbausituation des Lüfterrades (15) im Bereich des Gebläseunterteiles (17) erkennbar. Gemäß der dargestellten Ausführungsform sind zusätzliche Fördersegmente (23) verwendet, die jeweils mit einem Abstand zueinander entlang von spiralförmigen Linien ausgehend von der Einströmöffnung (25) zu einem Umfang der Tragplatten (21,22) verlaufen. Hierdurch wird eine verbesserte Förderleistung erreicht und eine Strömungsrichtung ausgehend von der Einströmöffnung (25) in eine radial nach außen weisende Richtung unterstützt.

Korrespondierend zu den Befestigungsstegen (32) im Bereich des Luftleitelementes (1) ist das Gehäuseunterteil (17) mit Gegenelementen (33) versehen. Das Gehäuseunterteil (17) trägt einen Ausströmstutzen (34), der im wesentlichen in tangentialer Richtung an das Gebläseunterteil (17) angeflanscht ist. Im Bereich seiner dem Lüfterrad (15) abgewandten Ausdehnung trägt der Ausströmstutzen (34) ein Kopplungsstück (35) zur Verbindung mit einem Versorgungsschlauch, der an einer Beatmungsmaske befestigt ist.

## Patentansprüche

1. Luftzuführung für Gebläse (13) von Geräten zur Beatmung, wie zur Behandlung der Schlafapnoe, wobei unter Zwischenschaltung des Gebläses (13) ein Luftzuführkanal (3) mit einem Luftausgang verbunden ist, sowie bei der der Luftzuführkanal (3) in mindestens zwei Teilkanäle (5) aufgeteilt ist, die sich wenigstens über einen Teil der Längsausdehnung des Luftzuführkanals (3) in Richtung der Luftströmung erstrecken, **dadurch gekennzeichnet, daß** sich der Luftzuführkanal (3) mindestens bereichsweise relativ zu einem Zentralbereich (10) spiralförmig erstreckt und daß eine Ausströmöffnung (7) in einem Zentralbereich (10) des Luftleitelementes (1) derart angeordnet ist, daß die Ausströmöffnung (7) in einen zentralen Lüfterraum eines Lüfterrades (15) des Gebläses (13) mündet.

2. Luftzuführung nach Anspruch 1, **dadurch gekennzeichnet, daß** der Luftzuführkanal (3) mit einem luftdurchlässigen Material zur Bildung einer Vielzahl von Durchlässen in der Art von Teilkanälen ausgefüllt ist.

3. Luftzuführung nach Anspruch1, **dadurch gekennzeichnet, daß** mindestens drei Teilkanäle (5) in einer räumlichen Richtung nebeneinander angeordnet sind.

4. Luftzuführung nach einem der Ansprüche 1 bis 3, **dadurch gekennzeichnet, daß** die Teilkanäle (5) in unterschiedlichen räumlich getrennten Ebenen angeordnet sind.

5. Luftzuführung nach einem der vorhergehenden Ansprüche, **dadurch gekennzeichnet, daß** die Teilkanäle (5) mindestens bereichsweise ein poröses Material aufnehmen.

6. Luftzuführung nach Anspruch 5, **dadurch gekennzeichnet, daß** die Teilkanäle (5) als poröses Material ein grob gesintertes Material aufnehmen.

7. Luftzuführung nach Anspruch 5, **dadurch gekennzeichnet, daß** die Teilkanäle (5) als poröses Material einen Schaumstoff aufnehmen.

8. Luftzuführung nach einem der vorhergehenden Ansprüche, **dadurch gekennzeichnet, daß** die Teilkanäle (5) mindestens teilweise im Eintrittsbereich eine Querschnittserweiterung aufweisen.

9. Luftzuführung nach einem der vorhergehenden Ansprüche, **dadurch gekennzeichnet, daß** sich die Teilkanäle (5) mindestens bereichsweise konzentrisch zu einem Mittelpunkt (30) des Luftleitelementes (1) sowie entlang eines wesentlichen Teiles ihres Verlaufes in Strömungsrichtung mit näherungsweise konstanter Querschnittfläche erstrecken.

10. Luftzuführung nach einem der vorhergehenden Ansprüche, **dadurch gekennzeichnet, daß** das Lüfterrad (15) des Gebläses (13) Fördersegmente (23) derart aufweist, daß eine Strömungsrichtung relativ zu einer Mittelachse des Lüfterrades (15) nach außen weisend vorgegeben ist.

## Claims

1. Air supply device for fans (13) of respirators, such as for treating sleep apnoea, wherein an air supply duct (3) is connected to an air outlet, with the fan (13) being disposed in between, and in which the air supply duct (3) is divided into at least two partial ducts (5) which extend at least over a part of the longitudinal extent of the air supply duct (3) in the direction of the air flow, **characterised in that** the air supply duct (3) extends spirally relative to a central region (10), at least in certain regions, and that an outflow opening (7) is disposed in a central region (10) of the air guide element (1) such that the outflow opening (7) leads into a central ventilator space of an impeller (15) of the fan (13).

2. Air supply device according to Claim 1, **characterised in that** the air supply duct (3) is filled with an air-permeable material for forming a plurality of passages in the manner of partial ducts.

3. Air supply device according to Claim 1, **characterised in that** at least three partial ducts (5) are disposed side-by-side in one spatial direction.

4. Air supply device according to any one of Claims 1 to 3, **characterised in that** the partial ducts (5) are disposed in different planes which are separated in space.

5. Air supply device according to any one of the preceding Claims, **characterised in that** the partial ducts (5) accommodate a porous material, at least in certain regions.

6. Air supply device according to Claim 5, **characterised in that** the partial ducts (5) accommodate a coarsely sintered material as porous material.

7. Air supply device according to Claim 5, **characterised in that** the partial ducts (5) accommodate a foamed material as porous material.

8. Air supply device according to any one of the preceding Claims, **characterised in that** the partial ducts (5) have a cross-sectional widening in the inlet region, at least in part.

9. Air supply device according to any one of the preceding Claims, **characterised in that** the partial ducts (5) extend concentrically about a centre point (30) of the air guide element (1), at least in certain regions, and along a substantial part of their path in the flow direction with an approximately constant cross-sectional area.

10. Air supply device according to any one of the preceding Claims, **characterised in that** the impeller (15) of the fan (13) has delivery segments (23) such that a flow direction which points outwardly relative to a centre axis of the impeller (15) is predetermined.

## Revendications

1. Amenée d'air pour soufflante (13) d'appareils pour la respiration artificielle, comme pour le traitement de l'apnée du sommeil, où un canal d'amenée d'air (3) est relié à une sortie d'air en intercalant la soufflante (13), ainsi que dans laquelle le canal d'amenée d'air (3) est divisé en au moins deux canaux partiels (5) qui s'étendent sur au moins une partie de la longueur du canal d'amenée d'air (3) dans la direction du courant d'air, **caractérisé en ce que** le canal d'amenée d'air (3) s'étend en forme de spirale au moins partiellement, par rapport à une zone centrale (10) et **en ce qu'**un orifice d'évacuation (7) est disposé dans une zone centrale (10) de l'élément de canalisation d'air (1), de sorte que l'orifice d'évacuation (7) débouche dans un espace central de ventilation, d'une roue de ventilation (15) de la soufflante.

2. Amenée d'air suivant la revendication 1, **caractérisée en ce que** le canal d'amenée d'air (3) est rempli d'un matériau perméable à l'air pour former un certain nombre de passages du type de canaux partiels.

3. Amenée d'air suivant la revendication 1, **caractérisée en ce qu'**au moins trois canaux partiels (5) sont disposés l'un à côté de l'autre en une direction spatiale.

4. Amenée d'air suivant l'une quelconque des revendications 1 à 3, **caractérisée en ce que** les canaux partiels (5) sont disposés dans différents plans spatiaux.

5. Amenée d'air suivant l'une quelconque des revendications précédentes, **caractérisée en ce que** les canaux partiels (5) présentent au moins partiellement, un matériau poreux.

6. Amenée d'air suivant la revendication 5, **caractérisée en ce que** les canaux partiels (5) présentent comme matériaux poreux, un matériau grossièrement fritté.

7. Amenée d'air suivant la revendication 5, **caractérisée en ce que** les canaux partiels (5) présentent comme matériaux poreux, une mousse.

8. Amenée d'air suivant l'une quelconque des revendications précédentes, **caractérisée en ce que** les canaux partiels (5) présentent au moins partiellement dans la zone d'entrée, un élargissement de section.

9. Amenée d'air suivant l'une quelconque des revendications précédentes, **caractérisée en ce que** les canaux partiels (5) s'étendent au moins par zone, de manière concentrique à un point central (30) de l'élément de canalisation d'air (1), ainsi que le long d'une partie importante de son tracé avec une section presque constante.

10. Amenée d'air suivant l'une quelconque des revendications précédentes, **caractérisée en ce que** la roue de ventilation (15) de la soufflante (13) présente des segments de refoulement (23) de sorte qu'une direction d'écoulement est définie dirigée vers l'extérieur, par rapport à un axe central de la roue de ventilation (15).
